# EUROPÄISCHE PATENTSCHRIFT

(11) **EP 2 155 304 B1**
(45) Veröffentlichungstag und Bekanntmachung des Hinweises auf die Patenterteilung: **11.09.2013**
(21) Anmeldenummer: 08758165.8
(22) Anmeldetag: 05.06.2008
(51) Int. Cl.: A61M 5/30, A61M 5/24, A61M 5/19, A61M 5/178

(54) **VORRICHTUNG ZUR AUFBEWAHRUNG UND APPLIKATION VON WIRKSTOFFEN**
DEVICE FOR STORING AND ADMINISTERING ACTIVE SUBSTANCES
SYSTÈME DE CONSERVATION ET D'ADMINISTRATION DE SUBSTANCES ACTIVES

(30) Priorität: 09.06.2007 DE 102007026752
(43) Veröffentlichungstag der Anmeldung: 24.02.2010
(73) Patentinhaber: Schimbach, Martina, 55218 Ingelheim (DE)
(72) Erfinder: Schimbach, Martina, 55218 Ingelheim (DE)
(86) Internationale Anmeldenummer: PCT/DE2008/000937
(87) Internationale Veröffentlichungsnummer: WO 2008/151606

(56) Entgegenhaltungen:
- DE-A1- 2 158 910
- US-A1- 2003 153 900

## Beschreibung

Die Erfindung bezieht sich auf eine Vorrichtung zur Aufbewahrung und Applikation von Wirkstoffen mit mehreren Reservoirs für jeweils mindestens einen Wirkstoff und einer nadellosen Injektionseinrichtung zur Zuführung eines oder mehrerer Wirkstoffe an einen Nutzer.

Eine derartige Vorrichtung ist aus der DE 21 58 910 A1 bekannt.

Zur Gesundheitsvorsorge ist es bekannt, an sich beliebige Wirkstoffe gekühlt zu lagern, um ihre jeweilige Wirksamkeit auch über einen längeren Zeitraum aufrecht zu erhalten. Hierzu ist üblicherweise die Bevorratung dieser Wirkstoffe in einem Kühlschrank notwendig. Wirkstoffe müssen in der Regel in zeitlich festgelegten Abständen eingenommen werden oder beispielsweise bei Diabetikern in Abhängigkeit eines Blutzuckerspiegels, der unter anderem durch die Einnahme von Nahrungsmitteln verändert wird. Dazu kann ein solcher Patient eine so genannte "Diabetiker-Uhr" mit sich führen.

Im Weiteren ist lediglich ein Reservoir mit einem Wirkstoff vorhanden, weshalb die Zuführung mehrerer Wirkstoffe nur durch den Gebrauch mehrerer entsprechender Vorrichtungen sichergestellt ist.

Es ist Aufgabe der Erfindung, eine Vorrichtung zur vereinfachten Aufbewahrung und Applikation von mehreren Wirkstoffen zu schaffen, die einfach handhabbar ist und es einem Nutzer ermöglicht, sich unabhängig und frei zu bewegen.

Erfindungsgemäß wird die Aufgabe dadurch gelöst, dass die Reservoirs sternförmig mit einer Stirnseite in Richtung eines Zentrums der Vorrichtung weisend angeordnet sind und ein Mechanismus zum Öffnen und Schließen der Reservoirs in der Vorrichtung gelagert ist

Ein wesentlicher Gedanke der Erfindung besteht darin, dass die Vorrichtung mit einem Reservoir für an sich beliebige Wirkstoffe in einer Form, Größe und Gewicht entsprechend einer handelsüblichen Armbanduhr ausgeführt ist. Somit können die Reservoirs oder Vorratsbehälter mit den Wirkstoffen, wie beispielsweise Enzyme, Propolis, Säuren, Basen, Vitamine, Mineralstoffe, Vitalstoffe und dergl., von einem Nutzer stets bequem mit sich geführt werden. Mittels der nadellosen Injektionseinrichtung kann ein im Reservoir vorrätig gehaltener Wirkstoff an den Nutzer abgegeben werden, wobei selbstverständlich auch eine Kombination von Wirkstoffen aus unterschiedlichen Reservoirs verabreichbar ist. Selbstverständlich kann die Vorrichtung mit einem ästhetisch ansprechenden Design versehen sein.

Damit die Vorrichtung bei der Unterbringung einer Vielzahl von Reservoirs einen kompakten Aufbau aufweist, sind die Reservoirs sternförmig mit einer Stirnseite in Richtung des Zentrums der Vorrichtung weisend angeordnet und ein, insbesondere rechnergesteuerter, Mechanismus zum Öffnen und Schließen der Reservoirs ist vorzugsweise motorisch drehbar in der Vorrichtung gelagert. Über den insbesondere motorisch angetriebenen Mechanismus zum Öffnen und Schließen der Reservoirs lässt sich die Menge des aus dem jeweiligen Reservoir ausgetragenen Wirkstoffs relativ exakt bestimmen.

In Ausgestaltung umfasst der Mechanismus zwei jeweils eine Öffnung aufweisende konzentrisch gelagerte Ringe, die sowohl relativ zueinander als auch gegenüber den Reservoirs drehbar sind, wobei die Öffnung des inneren Ringes mit der nadellosen Injektionseinrichtung gekoppelt ist. Um einen bestimmten Wirkstoff zu verabreichen, dreht sich der äußere Ring mit seiner Öffnung zu dem entsprechenden Reservoir und der innere Ring gibt nach einer entsprechenden Drehbewegung, beispielsweise für einen bestimmten Zeitraum, die Öffnung des äußeren Ringes frei, worauf der entsprechende Wirkstoff zu der nadellosen Injektionseinrichtung gelangt. Dieser Vorgang der Wirkstoffentnahme kann an verschiedenen Reservoirs wiederholt werden, um eine gewünschte Wirkstoffkombination zusammenzustellen. Zur Applikation oder Zuführung der Wirkstoffe an einen Nutzer dient die nadellose Injektionseinrichtung. Nadellose Injektionseinrichtungen sind dem Fachmann in einer Vielzahl bekannt. Diese Injektionseinrichtungen transportieren beispielsweise flüssige Wirkstoffe unter Anwendung von Federkraft, die einen Hochdruck erzeugt, durch die Haut des Nutzers.

Nach einer Weiterbildung besteht jedes Reservoir aus einem flexiblen Material, insbesondere Silikon, und jedes einzelne Reservoir ist separat oder alle Reservoirs sind gemeinsam druckbeaufschlagt. Zweckmäßigerweise ist zur Druckbeaufschlagung der Reservoirs ein flexibler Druckring in der Vorrichtung angeordnet, der die Reservoirs mit einer Kraft zur Erhöhung des Innendruckes betätigt. Der Druckring kann beispielsweise mit Luft gefüllt sein oder aufgrund seiner elastischen Materialeigenschaften die relativ weichen Reservoirs zusammendrücken.

Damit eine definierte Menge an Wirkstoff aus den Reservoir ausgetragen wird, ist vorzugsweise mindestens einem Reservoir und/oder dem Druckring ein mit einer Rechnersteuerung verbundener Druck- bzw. Lagesensor zugeordnet, wobei die Rechnersteuerung mindestens einen Speicherbaustein und/oder eine Schnittstelle umfasst und mit einer Eingabe- und/oder Ausgabeeinrichtung verbunden ist, die vorzugsweise mindestens einen Tastschalter und mindestens eine Kontrollleuchte umfasst. Über den Tastschalter bzw. die Schnittstelle lassen sich Daten bezüglich der in den Reservoirs vorhandenen Wirkstoffe, deren zu applizierende Menge sowie die Uhrzeit, zu der die Wirkstoffe verabreicht werden sollen, eingeben und in dem Speicherbaustein insbesondere überschreibbar hinterlegen. Selbstverständlich ist es möglich, dass der Rechner den Mechanismus zum Öffnen und Schließen der Reservoirs und/oder die nadellose Injektionseinrichtung steuert. Für Eingaben direkt an der Vorrichtung steht der mindestens eine Tastschalter zur Verfügung. Die Ausgabeeinrichtung, die beispielsweise als eine Mehrfarbige LED oder eine Sprachausgabeeinrichtung realisiert ist, informiert den Nutzer der Vorrichtung beispielsweise über den Füllzustand der Reservoirs. Auf den Speicherbaustein können neben einer individuellen Zusammensetzung zu verabreichender Wirkstoffe weitere den Nutzer betreffende Daten gesichert gespeichert sein, die neben Name, Alter, Adresse und Geschlecht beispielsweise Informationen zu Body Mass Index, Hobby, Kalorien-Verbrauch, Gesundheitszustand, Medikamenteneinnahme und dergl. umfassen. Aufgrund der Rechnersteuerung der Vorrichtung ist es beispielsweise möglich, über einen Lackmusstreifen einen pH-Wert des Speichels des Nutzers zu erfassen und in die Vorrichtung einzugeben, die daraufhin entsprechende Wirkstoffe freisetzt. Ebenso können Wirkstoffe in einer zeitlich gewünschten Abfolge freigesetzt werden. Des Weiteren ist es möglich, dass weitere biometrische Daten des Nutzers, beispielsweise Puls, Hauttemperatur und dergl., über geeignete Sensoren von der Vorrichtung erfasst und überwacht werden.

In weiterer Ausgestaltung sind die Reservoirs einzeln oder gemeinsam austauschbar oder mit einer Einheit zum Befüllen mit beliebigen Wirkstoffen versehen, wobei die Einheit zum Befüllen ein von der Öffnung des inneren Ringes abgehendes, sich in Richtung des Zentrums des Ringes ersteckendes Röhrchen umfasst, das vorzugsweise endseitig einen teleskopierbaren Stift, insbesondere aus Silikon, mit mindestens einer Öffnung zum Hindurchtreten eines Wirkstoffs umfasst. Der Stift der Einheit zum Befüllen kann ausgefahren werden, um den Wirkstoff aus einem Vorratsbehälter durch Poren, bzw. Öffnungen, in das entsprechende Reservoir zu leiten. Werden vorzugsweise die Reservoirs der Vorrichtung, wie im Folgenden beschrieben, an einer Nachfüllstation aufgefüllt, ist die Einheit zum Befüllen natürlich mit dem Rechner verbunden. Zu Beginn des Nachfüllens wird der Mechanismus zum Öffnen und Schließen der Reservoirs in eine bestimmte Stellung verfahren und Informationen bezüglich der in der Vorrichtung enthaltenen Wirkstoffe bzw. der Position der Reservoirs ausgetauscht, um sicherzustellen, dass die in der Vorrichtung vorhandenen Reservoirs auch mit entsprechendem Wirkstoff befüllt werden. Die korrekte Position des teleskopartigen Stifts und/oder des Mechanismus zum Öffnen und Schließen und/oder der Füllstand der Reservoirs werden sensorisch überwacht.

Zweckmäßigerweise ist das Röhrchen mit der nadellosen Injektionseinrichtung verbunden und sowohl die Einrichtung zur Zuführung des Wirkstoffs oder einer Kombination von Wirkstoffen aus mehreren Reservoirs an einen Nutzer als auch die Einheit zum Befüllen des Reservoirs sind bei einer Nichtbenutzung von einer verlagerbaren Blende abgedeckt. Die Blende kann beispielsweise beim Ein- bzw. Ausschalten der Vorrichtung oder beim Betätigen der Einrichtung zur Zuführung des Wirkstoffs bzw. der Einheit zum Befüllen des Reservoirs selbsttätig geöffnet werden. Natürlich sind alle Komponenten und Bestandteile der Vorrichtung in einem Gehäuse angeordnet, um insbesondere die Reservoirs vor äußeren Einflüssen zu schützen.

Vorzugsweise ist eine Abgabe der Wirkstoffe insbesondere personalisierbar vorgebbar. Um einen Missbrauch der Vorrichtung zu vermeiden, ist diese personalisierbar. Hierzu kann unter anderem ein Fingerabdrucksensor, ein Iris-Scanner oder dergl. vorgesehen sein, um die Identität des Nutzers und dessen Berechtigung festzustellen. In gleicher Weise kann ein Codewort, eine PIN oder dergl. eingegeben werden oder die Vorrichtung verfügt über eine Sprach- und Stimmerkennung, um einen Wirkstoff nur an einen berechtigten Nutzer abzugeben. Hierbei kann die Vorrichtung auch derart ausgebildet sein, dass eine Freigabe, insbesondere an Kinder oder gebrechliche Personen, durch eine autorisierte Person erfolgt.

Bevorzugt ist die Vorrichtung in Form, Größe und Gewicht entsprechend einer Armbanduhr ausgeführt, ein Kältemittelspeicher, insbesondere für flüssiges Helium, und gegebenenfalls Solarzellen für die Energieversorgung sind vorgesehen, wobei die Vorrichtung beispielsweise eine Schmutz abweisende Oberfläche, insbesondere entsprechend dem Lotus-Effekt, aufweist. Durch den an sich beliebig ausgestaltbaren Kältemittelspeicher werden die Wirkstoffe gekühlt und deren Wirksamkeit aufrecht erhalten. Zweckmäßigerweise ist der Kältemittelspeicher in Form eines Gefäßes für flüssiges Helium ausgebildet, um eine maximale Kühlkapazität zu erhalten. Dabei kann zwischen einem umgebenden Gehäuse der Vorrichtung und dem eigentlichen Kältemittelspeicher ein räumlicher Abstand, beispielsweise 0,5 mm, gewählt werden, um ein angenehmes Tragegefühl der armbanduhrartigen Vorrichtung zu erhalten. Bevorzugt ummantelt oder umhüllt der Kältemittelspeicher in Form einer Vakuumflasche für flüssiges Helium die Reservoirs allseitig, um sie zu kühlen. Am Kältemittelspeicher ist sowohl ein Einfüllstutzen für das flüssige Helium als auch ein Überdruckventil vorhanden, um verdampftes Helium ableiten zu können. Zur Energieversorgung kann die Vorrichtung mit einer Batterie oder einem Akkumulator versehen sein, bevorzugt ist sie jedoch mit Solarzellen zur Erzeugung des benötigten elektrischen Stroms ausgerüstet. Beschichtungen von Oberflächen zur Erzielung des Lotus-Effektes sind aus dem Stand der Technik bekannt.

Zur Benutzung der Vorrichtung wird diese durch die Beaufschlagung einer EIN-Taste eingeschaltet und es erfolgt über die Schnittstelle ein Abgleich zwischen Daten der Vorrichtung und Daten eines Zentralrechners, der beispielsweise in einer Heim-Nachfüllstation integriert ist, zur aktuellen Vitalstoff-Tagesbedarf-Ermittlung des Nutzers der Vorrichtung, der insbesondere durch ein Sprach-/StimmenErkennungsprogramm zu identifizieren ist. Es erfolgt in bestimmten Zeitintervallen die Berechnung des Leistungsumsatzes des Nutzers auf Basis eines Grundumsatzes, d.h. dem Kalorienverbrauch für Körperfunktionen. Bei älteren Nutzern erfolgt die Kontrolle des Cholesterin-/Kalzium-/Vitamin D-Haushalts. Ein rotes Lichtsignal der ampelartig ausgestalteten Ausgabeeinrichtung gibt beispielsweise einen Hinweis darauf, dass zu wenig oder zu viel Flüssigkeit zugeführt wurde bzw. eine Unter- oder Überdeckung des Tagesbedarfs von bestimmten Stoffen besteht. Die Vorrichtung gibt rechnergesteuert bestimmte Wirkstoffe ab, um entsprechende Defizite zu beseitigen. Im Weiteren können über eine entsprechende Ausgabeeinrichtung verhaltensbezogene Vorschläge ausgegeben werden, beispielsweise Hinweise auf Lebensmittel und/oder Kräuter, die zu meiden sind. Bei einem grünen Lichtsignal erfolgt keine Aktion der Vorrichtung, da keine bedenklichen Daten vorliegen. Zur Genesungsunterstützung gibt die Vorrichtung in einer bestimmten Sequenz die geeigneten Wirkstoffe in geeigneter Menge ab und informiert über geeignete Heilkräuter. Sobald die Vorrichtung z.B. zum Schlafengehen, abgelegt wird, wird sie durch das Öffnen eines Verschlusses des Armbandes deaktiviert.

Um unter anderem auf Reisen die Vorrichtung wieder zu befüllen, ist nach einer Weiterbildung ein System vorgesehen, das zum einen eine vorstehend beschriebene Vorrichtung und zum anderen eine Nachfüllstation umfasst. Eine solche Nachfüllstation mit entsprechend großen Vorratsbehältern für verschiedene Wirkstoffe kann sowohl öffentlich zugänglich als auch als private Nachfüllstation ausgebildet sein. In der Nachfüllstation sind unterschiedliche Reservoirs für verschiedene Wirkstoffe vorgesehen, die insbesondere alle aus Silikon bestehen und ebenfalls gekühlt sind und die beispielsweise von einem Betreiber regelmäßig mit Wirkstoffen nachgefüllt werden. Selbstverständlich müssen die Wirkstoffe hierzu jeweils geeignet luft- und/oder lichtdicht abgepackt sein. Das Nachfüllen einer privaten Nachfüllstation erfolgt durch den Nutzer selbst, der hierfür entsprechende Reservoirs der jeweils gewünschten Wirkstoffe einkaufen und somit seine Nachfüllstation nachfüllen kann. Den Reservoirs der Nachfüllstation ist die Einrichtung zur Abgabe von Wirkstoffen an die Vorrichtung zugeordnet, die insbesondere zwei, jeweils eine Öffnung aufweisende konzentrisch gelagerte Ringe umfasst, die sowohl relativ zueinander als auch gegenüber den Reservoirs drehbar sind, wobei der innere Ring mit einer Leitung zur Kopplung mit der Vorrichtung in Verbindung steht. Die Leitung ist mit einem verschließbaren Auslass zum Anschluss an die Einheit zum Befüllen der Vorrichtung, insbesondere an deren teleskopierbaren Stift versehen.

Zum Nachfüllen wird die armbanduhrartige Vorrichtung in einer geeigneten Halterung an der Nachfüllstation platziert, und der verschließbare Auslass der Nachfüllstation an den teleskopartigen Stift der Vorrichtung angekoppelt, so dass das Reservoir in der Vorrichtung aufgefüllt werden kann. Eine entsprechende Bezahlung kann bargeldlos oder durch Münzeinwurf bzw. Geldscheineingabe erfolgen.

Da die Vorrichtung individuell zu befüllen ist, ist insbesondere bei einer öffentlichen Nachfüllstation eine Personalisierung vorteilhaft, um ein falsches Nachfüllen des Reservoirs in der Vorrichtung zu vermeiden. Vorzugsweise ist die Nachfüllstation rechnergesteuert und kommuniziert über eine Schnittstelle mit der Vorrichtung oder einem Zentralrechner. Demnach kann die Nachfüllstation feststellen, mit welchen Wirkstoffen und in welcher Zusammensetzung die Vorrichtung zu befüllen ist.

Damit sichergestellt ist, dass die Vorrichtung bzw. deren Reservoir mit der individuell festgelegten Mischung von Wirkstoffen befüllt ist, umfasst zweckmäßigerweise die Nachfüllstation eine Entleereinrichtung für die Vorrichtung. Vor jedem Befüllen wird die Vorrichtung durch die Entleereinrichtung zunächst komplett geleert. Die Entleereinrichtung kann beispielsweise einen Entsorgungsbehälter für die Wirkstoffe aus der Vorrichtung enthalten. Im Weiteren kann zusätzlich oder alternativ eine Reinigungseinrichtung für die Vorrichtung und/oder die Einrichtung zur Abgabe von Wirkstoffen an die Vorrichtung vorgesehen sein.

Bevorzugt weist die Nachfüllstation eine mit dem Rechner verbundene Eingabe- und/oder Ausgabeeinrichtung auf. Bei der privaten Nachfüllstation zeigt beispielsweise ein grünes Lichtsignal, dass die Vorrichtung aufgeladen ist.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispiels mit Bezugnahme auf die Zeichnungen näher erläutert. Es zeigt:

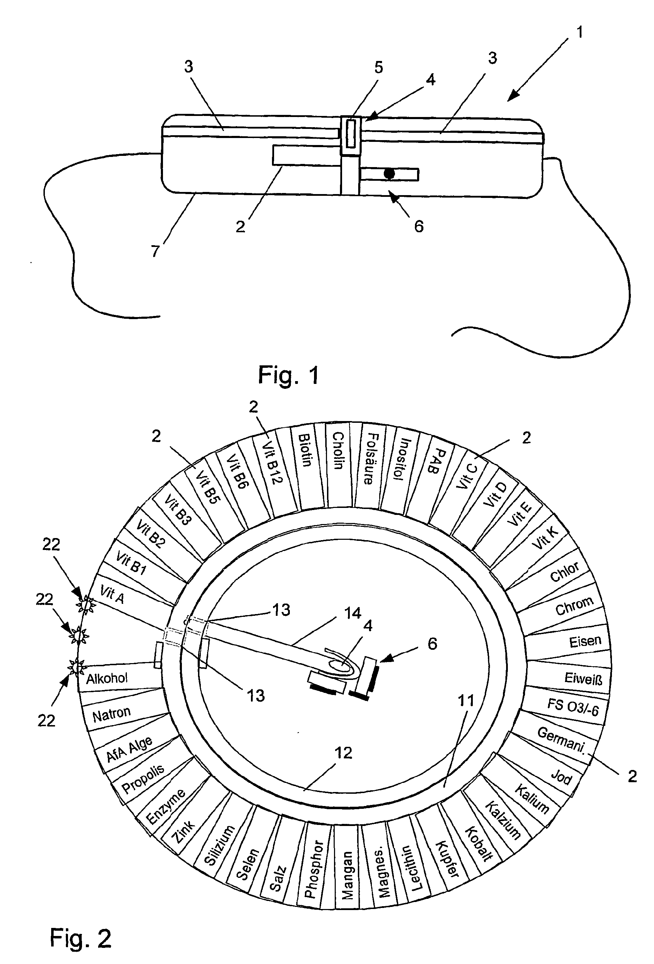

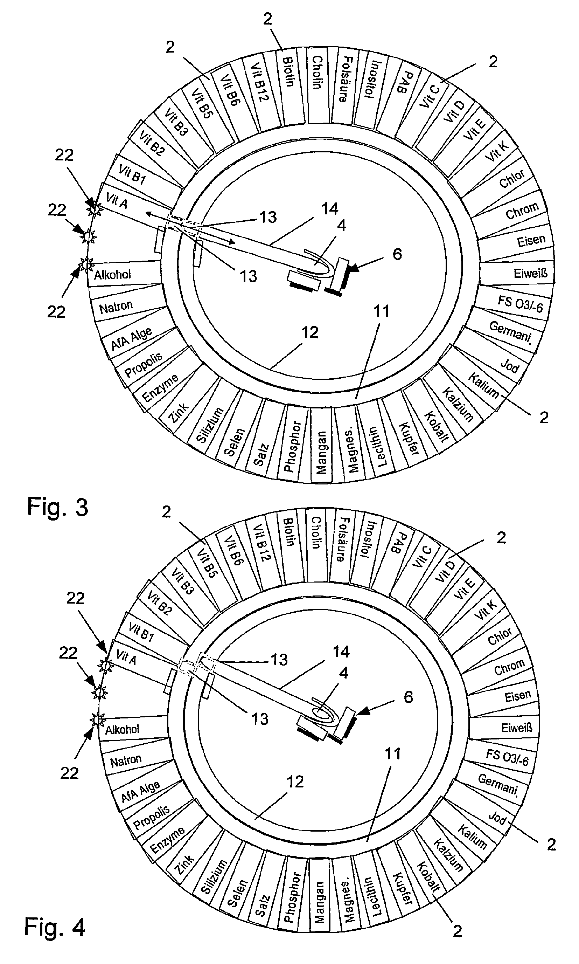

- Fig. 1.: eine erfindungsgemäße Vorrichtung in schematischer Darstellung,

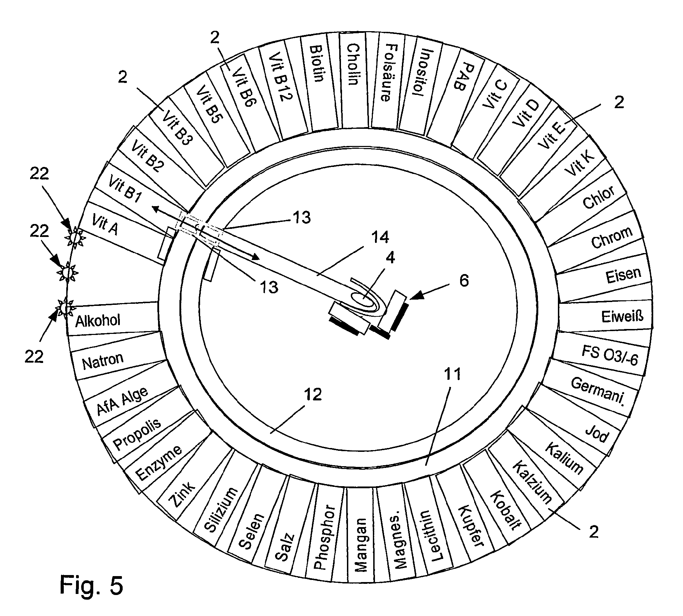

- Fig. 2. bis Fig. 5.: schematische Schnittdarstellungen durch die Vorrichtung nach Fig. 1 in unterschiedlichen Betriebszuständen,

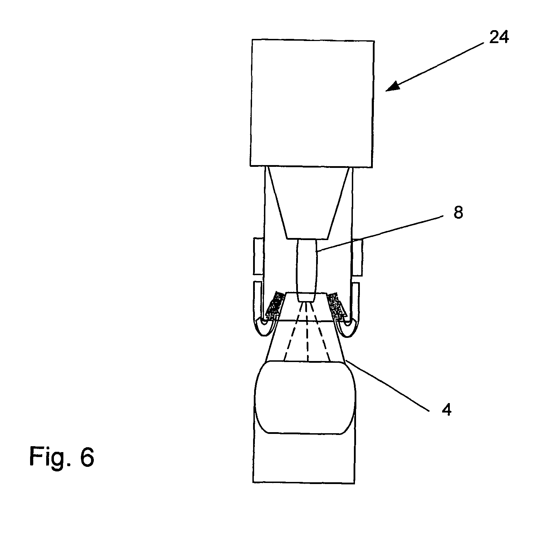

- Fig. 6.: eine schematische Teildarstellung eines Systems, umfassend die Vorrichtung nach Fig. 1 und eine Nachfüllstation und

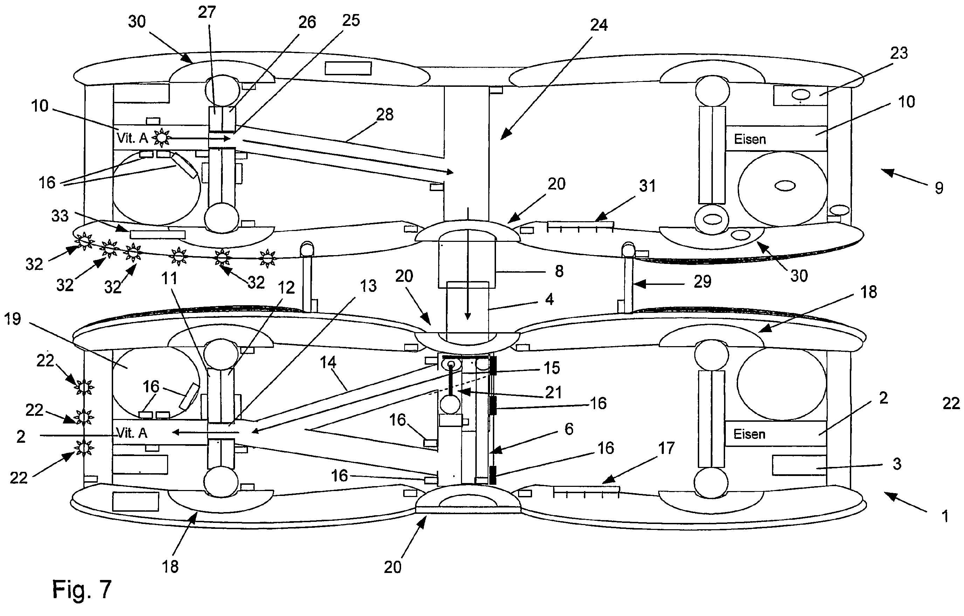

- Fig. 7.: eine schematische Schnittdarstellung durch das System nach Fig. 6.

Die Vorrichtung 1 zur Aufbewahrung und Applikation von Wirkstoffen verfügt über mehrere Reservoirs 2 aus Silikon, in denen an sich beliebige Wirkstoffe, wie Enzyme, Propolis oder dergl., lebensmittel- bzw. arzneimittelecht bevorratet werden. Um die Wirksamkeit der Wirkstoffe dauerhaft zu erhalten, ist der Vorrichtung 1 ein Kältemittelspeicher 3, insbesondere in Form einer Vakuumflasche für Helium, zugeordnet.

Zum Nachfüllen des Reservoirs 2 dient ein teleskopierbarer Stift 4, der unter Luftdruck bzw. Vakuum-Beaufschlagung oder mechanisch betätigt aus- und eingefahren werden kann, wobei der Stift 4 aus Silikon bestehen kann und eine Öffnung 5 für den Wirkstoff aufweist. Liegt der ausgefahrene Stift 4 an einem dazu korrespondierenden, beispielsweise teleskopierbaren, Auslass 8 einer öffentlichen oder privaten Nachfüllstation 9 an, so können die Wirkstoffe aus einem Reservoir 10 der Nachfüllstation 9, die insbesondere bezüglich der Anordnung der Reservoirs 10 in weiten Teilen übereinstimmend mit der Vorrichtung 1 ausgebildet sein kann, in das Reservoir 2 der Vorrichtung 1 übertreten, um diese wieder zu befüllen. Hierzu ist die Vorrichtung 1 insbesondere personalisiert, um einen Missbrauch der Vorrichtung 1 zu vermeiden.

Zur Abgabe von Wirkstoffen an einen Nutzer ist eine Einrichtung zur Zuführung des Wirkstoffs an einen Nutzer als eine nadellose Injektionseinrichtung 6 ausgebildet, die eine Federmechanik umfasst, um Wirkstoff aus den Reservoir 2 mittels hohem Druck durch die Haut des Nutzers zu befördern, wo der Wirkstoff seine Wirkung entfalten kann.

Die Vorrichtung 1 ist mit einem ansprechenden Gehäuse 7 nach Art einer Armbanduhr mit zugehörigem Armband versehen, um ein ästhetisches Äußeres zu erhalten.

Die Reservoirs 2 sind sternförmig mit einer Stirnseite in Richtung des Zentrums der Vorrichtung 1 weisend angeordnet und ein rechnergesteuerter Mechanismus zum Öffnen und Schließen der Reservoirs 2 in Form von zwei motorisch drehbaren Ringen 11, 12 mit jeweils einer Öffnung 13 ist in der Vorrichtung 1 gelagert. Die konzentrisch gelagerten Ringe 11, 12 sind sowohl relativ zueinander als auch gegenüber den Reservoirs 2 in entsprechenden Lagern 18 drehbar, wobei die Öffnung 13 des inneren Ringes 12 zum einen mit der nadellosen Injektionseinrichtung 6 und zum anderen mit einem sich in Richtung des Zentrums des Ringes 12 ersteckenden Röhrchen 14 gekoppelt ist, das endseitig den teleskopierbaren Stift 4 trägt. Ein Kanal 15 zum Durchfluss von Wirkstoffen in Richtung der nadellosen Injektionseinrichtung 6 oder des Stiftes 4 ist mit einer Ventileinrichtung 21 versehen. Die Lage der Ringe 11, 12 sowie des Stiftes 6, die nadellose Injektionseinrichtung 6, der Druck in dem Kanal 15, der Druck in den Reservoirs 2 und der Füllstand sowie die Temperatur in den Reservoirs 2 werden von Sensoren 16 überwacht, die mit einer Rechnersteuerung 17 verbunden sind. Zur Druckbeaufschlagung der Reservoirs 2 ist ein flexibler Druckring 19 in der Vorrichtung 1 angeordnet. Die Öffnungen für die Injektionseinrichtung 6 und den Stift 4, die selbstverständlich auch auf einer Seite der Vorrichtung angeordnet sein können, sind mittels einer Blende 20 verschlossen. Die Rechnersteuerung 17 ist mit Ein- und Ausgabeeinrichtungen 22 verbunden.

Im Weiteren ist eine Schnittstelle 29 zur mechanischen und/oder elektrischen Kopplung der Vorrichtung 1 mit der Nachfüllstation 9 vorgesehen, die ebenfalls die sternförmig angeordneten flexiblen Reservoirs 10 einen Kältemittelspeicher 23 und darüber hinaus eine Einrichtung 24 zur Abgabe von Wirkstoffen an die Vorrichtung 1 aufweist. Die Einrichtung 24 zur Abgabe von Wirkstoffen an die Vorrichtung 1 umfasst zwei jeweils eine Öffnung 25 aufweisende konzentrisch gelagerte Ringe 26, 27, die sowohl relativ zueinander als auch gegenüber den Reservoirs 10 motorisch drehbar sind, wobei der innere Ring 26 mit einer Leitung 28 zur Kopplung mit der Vorrichtung 1 über den Auslass 8 in Verbindung steht und die Ringe 26, 27 mittels Lager 30 in der Nachfüllstation 9 gehalten sind. Die Nachfüllstation 9 umfasst einen Rechner 31, an den Ein-/Ausgabeeinrichtungen 32 und Sensoren 33 angeschlossen sind, wobei die beispielsweise farbige LEDs umfassende Ein-/Ausgabeeinrichtungen 32 auch zur Darstellung von Zuständen der Nachfüllstation 9 bzw. dem Füllstand der Reservoirs 10 dient.

Befindet sich in zumindest einem Reservoir 2 der Vorrichtung 1 kein Wirkstoff, wird dies durch ein rotes Aufleuchten einer LED der Ein-/Ausgabeeinrichtung 22 dem Nutzer kenntlich gemacht und gleichzeitig die Vorrichtung 1 außer Betrieb genommen, da eine zuverlässige Versorgung des Nutzers mit Wirkstoffen nicht gewährleistet ist. Der Nutzer schließt daraufhin die Vorrichtung 1 an die Nachfüllstation 9 an und es beginnt entweder selbsttätig oder nach Beaufschlagung eines oder mehrerer Tastschalter der Ein-/ Ausgabeeinrichtungen 32, 22 der Nachfüllstation 9 bzw. der Vorrichtung 1 eine Datenübertragung bezüglich der in der Vorrichtung 1 vorhandenen und gegebenenfalls nachzufüllenden Wirkstoffe. Hierbei wird auch sichergestellt, dass sich die Öffnungen 13 der Ringe 11, 12 der Vorrichtung 1 und die Öffnungen 25 der Ringe 26, 27 der Nachfüllstation 9 in einer Ausgangslage befinden und nach dem Öffnen der dem Auslass 8 zugeordneten Blende 20 verfährt der Auslass 8 aus der Nachfüllstation 9, um den Stift 4 der Vorrichtung 1, der ebenfalls nach dem Öffnen der zugeordneten Blende 20 teleskopisiert wird, zu kontaktieren, damit Wirkstoff aus der Nachfüllstation 9 in das entsprechende Reservoir 2 der Vorrichtung 1 strömen kann. Hierzu werden die Ringe 11, 12 der Vorrichtung 1 rechnergesteuert mit ihren Öffnungen 13 vor das zu befüllende Reservoir 2 gedreht und die Ringe 26, 27 mit ihren Öffnungen 25 vor das entsprechende Reservoir 10 der Nachfüllstation 9. Der Füllstand der Reservoirs 2, 10 wird durch die zugeordneten Sensoren 16, 33 rechnergestützt überwacht. Nach dem Beenden des Nachfüllvorgangs, bei dem gegebenenfalls mehrere Reservoirs 2, 10 nacheinander angefahren werden, gehen die Nachfüllstation 9 und die Vorrichtung 1 in ihre jeweiligen Ausgangszustände zurück, in denen die Blenden 20 geschlossen sind.

Die Zusammensetzung und Verabreichung von Wirkstoffen erfolgt rechnergesteuert aufgrund gespeicherter Ausgangsdaten. Nachdem die zu verabreichenden Wirkstoffe durch das Öffnen und Schließen der Öffnungen 13 dosiert in der nadellosen Injektionseinrichtung 6 vorliegen, kann diese selbsttätig oder durch die Beaufschlagung einer Taste der Einund Ausgabeeinrichtung 22 ausgelöst werden.

Das Personalisieren der Vorrichtung 1 und der Nachfüllstation 9 erfolgt beispielsweise mittels der jeweiligen Ein-/ Ausgabeeinrichtung 22, 32. Über eine Bezahleinrichtung 33 kann Entgelt an einer öffentlich zugänglichen Nachfüllstation 9 entrichtet werden.

### Bezugszeichenliste

- 1.: Vorrichtung
- 2.: Reservoir v. 1
- 3.: Kältemittelspeicher v 1
- 4.: Stift
- 5.: Öffnung
- 6.: Injektionseinrichtung
- 7.: Gehäuse
- 8.: Auslass v 9
- 9.: Nachfüllstation
- 10.: Reservoir v 9
- 11.: Ring v 1
- 12.: Ring v 1
- 13.: Öffnung
- 14.: Röhrchen
- 15.: Kanal
- 16.: Sensor
- 17.: Rechnersteuerung
- 18.: Lager
- 19.: Druckring
- 20.: Blende
- 21.: Ventileinrichtung
- 22.: Ein/Ausgabeeinrichtungen
- 23.: Kältemittelspeicher v 9
- 24.: Einrichtung
- 25.: Öffnung
- 26.: Ring
- 27.: Ring
- 28.: Leitung
- 29.: Schnittstelle
- 30.: Lager
- 31.: Rechner
- 32.: Ein-/Ausgabeeinrichtung
- 33.: Bezahleinrichtung

## Patentansprüche

1. Vorrichtung zur Aufbewahrung und Applikation von Wirkstoffen mit mehreren Reservoirs (2) für jeweils mindestens einen Wirkstoff und einer nadellosen Injektionseinrichtung (6) zur Zuführung eines oder mehrerer Wirkstoffe an einen Nutzer, **dadurch gekennzeichnet, dass** die Reservoirs (2) sternförmig mit einer Stirnseite in Richtung eines Zentrums der Vorrichtung weisend angeordnet sind und ein Mechanismus zum Öffnen und Schließen der Reservoirs (2) in der Vorrichtung (1) gelagert ist.

2. Vorrichtung nach Anspruch 1, **dadurch gekennzeichnet, dass** der Mechanismus rechnergesteuert und vorzugsweise motorisch drehbar ist.

3. Vorrichtung nach Anspruch 1 oder 2, **dadurch gekennzeichnet, dass** der Mechanismus zwei jeweils eine Öffnung (13) aufweisende konzentrisch gelagerte Ringe (11, 12) umfasst, die sowohl relativ zueinander als auch gegenüber den Reservoirs (2) drehbar sind, wobei die Öffnung (13) des inneren Ringes (12) mit der nadellosen Injektionseinrichtung (6) gekoppelt ist.

4. Vorrichtung nach einem der Ansprüche 1 bis 3, **dadurch gekennzeichnet, dass** jedes Reservoir (2) aus einem flexiblen Material, insbesondere Silikon, besteht und jedes einzelne Reservoir (2) separat oder alle Reservoirs (2) gemeinsam druckbeaufschlagt sind.

5. Vorrichtung nach einem der Ansprüche 1 bis 4, **dadurch gekennzeichnet, dass** zur Druckbeaufschlagung der Reservoirs (2) ein flexibler Druckring (19) in der Vorrichtung (1) angeordnet ist, der die Reservoirs (2) mit einer Kraft zur Erhöhung des Innendruckes betätigt.

6. Vorrichtung nach einem der Ansprüche 1 bis 5, **dadurch gekennzeichnet, dass** mindestens einem Reservoir (2) und/oder dem Druckring (19) ein mit einer Rechnersteuerung verbundener Druck- bzw. Lagesensor (16) zugeordnet ist, wobei die Rechnersteuerung (17) mindestens einen Speicherbaustein und/oder eine Schnittstelle umfasst und mit einer Ein- und/oder Ausgabeeinrichtung (22) verbunden ist, die vorzugsweise mindestens einen Tastschalter und mindestens eine Kontrollleuchte umfasst.

7. Vorrichtung nach einem der Ansprüche 1 bis 6, **dadurch gekennzeichnet, dass** die Reservoirs (2) einzeln oder gemeinsam austauschbar oder mit einer Einheit zum Befüllen mit beliebigen Wirkstoffen versehen sind, wobei die Einheit zum Befüllen ein von der Öffnung (13) des inneren Ringes (12) abgehendes, sich in Richtung des Zentrums des Ringes (12) erstreckendes Röhrchen (14) umfasst, das vorzugsweise endseitig einen teleskopierbaren Stift (4), insbesondere aus Silikon, mit mindestens einer Öffnung (5) zum Hindurchtreten eines Wirkstoffs umfasst.

8. Vorrichtung nach einem der Ansprüche 1 bis 7, **dadurch gekennzeichnet, dass** das Röhrchen (14) mit der nadellosen Injektionseinrichtung (6) verbunden ist und sowohl die Einrichtung zur Zuführung des Wirkstoffs oder einer Kombination von Wirkstoffen aus mehreren Reservoirs (2) an einen Nutzer als auch die Einheit zum Befüllen des Reservoirs (2) bei einer Nichtbenutzung von einer verlagerbaren Blende (20) abgedeckt sind.

9. Vorrichtung nach einem der Ansprüche 1 bis 8, **dadurch gekennzeichnet, dass** eine Abgabe der Wirkstoffe insbesondere personalisierbar vorgebbar ist.

10. Vorrichtung nach einem der Ansprüche 1 bis 9, **dadurch gekennzeichnet, dass** die Vorrichtung (1) in Form, Größe und Gewicht entsprechend einer Armbanduhr ausgeführt ist, ein Kältemittelspeicher (3), insbesondere für flüssiges Helium, und gegebenenfalls Solarzellen für die Energieversorgung vorgesehen sind, wobei die Vorrichtung (1) insbesondere eine Schmutz abweisende Oberfläche entsprechend dem Lotus-Effekt aufweist.

11. System, bestehend aus einer Vorrichtung nach einem der Ansprüche 1 bis 10 und einer Nachfüllstation (9), wobei die Nachfüllstation (9) über mindestens ein Reservoir (10) für Wirkstoffe und einen Kältemittelspeicher (23) und über eine Einrichtung (24) zur Abgabe von Wirkstoffen an die Vorrichtung (1) verfügt.

12. System nach Anspruch 11, **dadurch gekennzeichnet, dass** jedes Reservoir (10) der Nachfüllstation (9) aus einem flexiblen Material, insbesondere aus Silikon, besteht und jedes einzelne Reservoir (2) separat oder alle Reservoirs (2) gemeinsam druckbeaufschlagt sind, wobei die Reservoirs (2) mit der Einrichtung (24) zur Abgabe von Wirkstoffen an die Vorrichtung (1) verbunden ist, die insbesondere zwei, jeweils eine Öffnung (25) aufweisende konzentrisch gelagerte Ringe (26, 27) umfasst, die sowohl relativ zueinander als auch gegenüber den Reservoirs (2) drehbar sind, wobei der innere Ring (26) mit einer Leitung (28) zur Kopplung mit der Vorrichtung (1) in Verbindung steht.

13. System nach Anspruch 12, **dadurch gekennzeichnet, dass** die Leitung (28) mit einem verschließbaren Auslass (8) zum Anschluss an die Einheit zum Befüllen der Vorrichtung, insbesondere an deren teleskopierbaren Stift (4) versehen ist.

14. System nach einem der Ansprüche 11 bis 13, **dadurch gekennzeichnet, dass** die Nachfüllstation (9) rechnerge steuert ist und über eine Schnittstelle mit der Vorrichtung (1) oder einem Zentralrechner kommuniziert, wobei der Rechner (31) der Nachfüllstation (9) insbesondere eine Eingabe- und/oder Ausgabeeinrichtung (32) aufweist und insbesondere bei einem elektrischen oder elektromechanischen Kontakt der Vorrichtung (1) mit der Nachfüllstation (9) ein automatischer Füllvorgang der Vorrichtung (1) beginnt und bei Vorliegen eines entsprechenden Sensorsignals der Vorrichtung (1) der Füllvorgang endet.

15. System nach einem der Ansprüche 11 bis 14, **dadurch gekennzeichnet, dass** die Nachfüllstation (9) eine Entleereinrichtung und/oder eine Reinigungseinrichtung für die Vorrichtung (1) und/oder die Einrichtung (24) zur Abgabe von Wirkstoffen an die Vorrichtung (1) umfasst.

## Claims

1. Device (1) for storage and administration of active ingredients, said the device (1) a plurality of reservoirs (2) for at least one active substance is comprising, said device (1) also a needleless injection device (6) for supplying one or more active substances to a user is comprising, said device (1) is **characterized in, that** all reservoirs (2) are star shaped arranged, with a frontal side all reservoirs (2) pointing toward a center of said device (1), within said device (1) a mechanism for opening and closing all reservoir (2) is mounted.

2. Device (1) according to claim 1, **characterized in, that** the mechanism is computer-controlled, and the mechanism is preferably motoric rotatable.

3. Device (1) according to any one of claims 1 to 2, **characterized in, that** the mechanism two concentrically mounted rings (11, 12) is comprising, both rings (11, 12) are an opening (13) comprise, the rings (11, 12) are rotatable relative to one another and are also rotatable relative to all reservoirs (2), wherein the opening (13) of the inner ring (12) via a tubule (14) coupled is with the needleless injection device (6).

4. Device (1) according to any one of claims 1 to 3, **characterized in, that** each reservoirs (2) are consisting of a flexible material, in particular of silicone, each single reservoir (2) with pressure is impinged separately, or all reservoirs (2) with pressure are being impinged together.

5. Device (1) according to one of claims 1 to 4, **characterized in**, wherein for pressurizing all reservoirs (2), a flexible pressure ring (19) in the device (1) is arranged which actuates all reservoirs (2) with a force, to increase their internal pressure.

6. Device (1) according to one of claims 1 to 5, **characterized in, that** at least to one reservoir (2) and / or the pressure ring (19) a pressure- and position sensor (16) is assigned, pressure- and position sensor (16) are associated to a computer-control (17), the computer-control (17) at least comprises a memory module and / or an interface, wherein said computer-control (17) is in connection with an input device and / or a dispensing means (22), the input device and f or dispensing means (22) preferably at least one pushbutton switch and at least one control light comprises.

7. Device (1) according to any one of claims 1 to 6, **characterized in, that** all reservoirs (2) individually or jointly, are interchangeable or all reservoirs (2) with a unit for filling them with any desired active substances are equipped, wherein said filling unit for filling, a tubule (14) comprises, what from the opening (13) of the inner ring (12) is branching off, the tubule (14) extends toward the center of the inner ring (12), the tubule (14) preferably ends to a telescopic pin (4), in particular of silicone, the telescopic pin (4) comprises at least one opening (5) for the passing through of an active ingredient.

8. Device (1) according to any one of claims 1 to 7, **characterized in, that** the tubule (14) with the needleless injection device (6) is connected, at non-usage the unit for supplying an active substance or a substance combination from a plurality of reservoirs (2) at one user as well as the entity for filling all the reservoirs (2) by a displacable aperture (20) are covered.

9. Device (1) according to one of claims 1 to 8, **characterized in, that** an ingredient delivery particularly personalizable specifiable is.

10. Device (1) according to any one of claims 1 to 9, **characterized in, that** the device (1) in form, size and weight is executed in accordance to a wrist watch, a coolant reservoir (3) especially for liquid helium is foreseen, and optionally through solar cells the energy supply is provided, wherein the device (1) in particular a dirt-repellent surface, corresponding to the Lotus effect, comprises.

11. System consisting of a device (1) according to one of claims 1 to 10 and a refillstation (9), wherein a refill station (9) at least comprises one reservoir (10) for active substances, and a refrigerant-reservoir (23) and it has a means (24) for delivery of active substances to the device (1).

12. System according to claim 11, **characterized in, that** each reservoir (10) of the refill station (9) is manufactured of a flexible material, in particular of silicone and each reservoir (10) is separately impinged with pressure respectively all reservoirs (10) together are impinged with pressure, while one of the reservoirs (10) being impinged with pressure, the reservoir (10) is in connection with an apparatus (24) via a tube (28) that delivers active substances to the device (1), said refill station (9) in particular two concentrically mounted rings (26, 27) comprises, each of these rings an opening (25) is comprising, the rings (26, 27) are rotatable relative to one another and relative to all reservoirs (10), the inner ring (26) is connected to a tube (28), the tube (28) is connected with the apparatus (24), the apparatus (24) is coupled to the device (1) while the apparatus (24) delivers active substances to the device (1).

13. System according to any one of claims 11 to 12, **characterized in, that** the apparatus (24) at its end is equipped with a closable outlet (20), during the filling process the mentioned apparatus (24) via a telescopable pin (8) connected is with the apparatus (6) for filling the device (1), in particular with the telescopable pin (4) of the device (1).

14. System according to any one of claims 11 to 13, **characterized in, that** the refill station (9) is computer-controlled, the refill station (9) communicates via an interface or a central computer with the device (1), the computer (31) of the refill station (9) in particular an input means and / or dispensing means (32) is comprises, the automatic refilling of the device (1) starts in particular with an electrical contact or an electro mechanical contact of the device (1) with said refill station (9), the refilling terminates with a corresponding sensor-signal to the device (1).

15. System according to any one of claims 11 to 14, **characterized in that** the refill station (9) comprises an emptying means and / or a cleaning means for emptying or cleaning of device (1) and / or the refill station (9).

## Revendications

1. Dispositif (1) pour le stockage et l'administration d'ingrédients actifs, la dispositif (1) une pluralité de réservoirs (2) pour au moins un ingrédient actif est comprend, ledit le dispositif (1) également comprenant l'une sans aiguille d'injection dispositif (6) pour fournir une ou plusieurs substances actives à la un utilisateur comprend, ledit dispositif (1) est a les **caractérisé en ce que** tous les réservoirs (2) sont disposées en forme d'étoile, ledit les tous réservoirs (2) sont l'orientée sont avec une côté frontal vers une centre dudit l'dispositif (1), en la dispositif (1) est à une mécanisme pour ouvrir et fermer les réservoirs (2) montée.

2. Dispositif (1) selon la revendication 1, **caractérisé en ce que** le mécanisme est commandé par un ordinateur, et le mécanisme est de préférence rotative par motor.

3. Dispositif (1) selon l'une quelconque des revendications 1 à 2, **caractérisé en ce que** le mécanisme comporte deux anneaux montés concentriquement (11, 12), chacun des deux anneaux (11, 12) l'une ouverture (13) comprend, des deux anneaux (11, 12) peuvent tourner par rapport à l'autre et sont aussi tourner par rapport à tous les réservoirs (2), l'ouverture (13) de la bague intérieure (12) au sujet d'un tubule (14) est couplé pour le d'injection sans aiguille moyen (6).

4. Dispositif (1) selon l'une des revendications 1 à 3, **caractérisé en ce que** chaque réservoir (2) est constitué matériau l'une flexible , sur silicone en particulier, et chaque unique réservoir (2) sont empiété séparément par pression, ou la totalité de réservoirs (2) en même temps sont empiété par pression sont.

5. Dispositif (1) selon l'une des revendications 1 à 4, caractérisé en ce, dans lequel, pour mettre sous pression à toutes d'réservoirs (2), une bague de pression flexible (19) dans le dispositif (1) est disposé qui actionne tous les réservoirs (2) avec une force, afin d'augmenter leur pression interne.

6. Dispositif (1) selon l'une des revendications 1 à 5, **caractérisé en ce, qu'**au moins d'une du réservoirs (2) et de la bague de pression (19) une capteur de pression et une capteur position (16) associé est, capteur de pression et capteur position (16) sont lié avec une de commande ordinateur (17) sont, dans lequel ledit de commande ordinateur (17) moins un module de mémoire et / ou une interface comprend est, ledit commande ordinateur (17) est lié avec d'une moyen d'entrée et / ou d'une moyen l'sortie (22) est, ledit les moyen d'entrée et / ou d'une moyen l'sortie (22) de préférence au moins un bouton-poussoir interrupteur et au moins une lumière de commande qui comprend.

7. Dispositif (1) selon l'une quelconque des revendications 1 à 6, **caractérisé en ce que** tous les réservoirs (2) sont individuellement ou conjointement sont interchangeables, ou tous les réservoirs (2) sont pour les remplir avec à tout désirée substances actives équipés avec d'une unité, le unité comprend une ouverture (13) dans la bague intérieure (12), à partir de l'ouverture (13) de la bague intérieure (12) un tubule (14) partir bifurque, la tubule (14) s'étend vers l'centre de la bague intérieure (12) s'étend, se termine le tubule (14) de préférence sur un télescopique broches (4), en particulier réalisé en silicone, ledit la télescopique broches (4) au moins une ouverture (5) pour le passage d'un ingrédient actif est comprend.

8. Dispositif (1) selon l'une quelconque des revendications 1 à 7, **caractérisé en ce que** le tubule (14) avec le d'injection sans aiguille moyen (6) est relié, entité pour l'administration d'un ingrédient actif ou une composition de principes actifs à partir d'une pluralité de réservoirs (2) à l'utilisateur, ainsi que unité pour le remplissage de tous les réservoirs (2) durant non l'utilisation avec par un déplaçable orifice (20) sont couverts.

9. Dispositif (1) selon l'une des revendications 1 à 8, **caractérisé en ce que**, en particulier le l'administration de substances actifs personnalisées spécifiable est pour la toutes les person.

10. Dispositif (1) selon l'une quelconque des revendications 1 à 9, **caractérisé en ce que** le dispositif (1) dans la forme, la taille et le poids en correspondant à une une montre-bracelet est exécutée, un réservoir de liquide de refroidissement (3) en particulier pour l'hélium liquide, est prévue et le cas échéant pour fourniture d'énergie cellules solaires sont prévu, dans lequel le dispositif (1) une surface résistant aux taches correspondant à l'effet lotus comprend en particulier.

11. Système, constitué d'un dispositif (1) selon l'une des revendications 1 à 10 et une recharge de station (9), dans lequel ladite comprend station de recharge (9) présente au moins un réservoir (10) pour les substances actives, et d'un réfrigérant à réservoirs (23) et une dispositif (24) pour la délivrance de substances actives à la l'appareil (1).

12. Système selon la revendication 11, **caractérisé en ce que** chaque réservoir (10) de la station de recharge (9) un matériau souple est fabriqué en, sur silicone en particulier, et particulier chaque réservoir (10) soit empiété séparément à la pression est, ou les totalité d'réservoirs (10) sont en même temps empiété par pression, tandis que l'un des réservoirs (10) est empiété par pression, est le réservoir (10) par d'un tube (28) en liaison avec de la dispositif (24) pour la délivrance de substances actives à la l'appareil (1), ledit poste de remplissage (9) comporte en particulier deux anneaux montés concentriquement (26, 27), chacun de ces anneaux une ouverture (25) est comprend, les anneaux (26, 27) sont peuvent tourner par rapport à l'autre et aussi en face à la tous les réservoirs (10), ledit la bague intérieure (26) par un tube (28) à la le dispositif (24) relié est, l'appareil (24) au cours de la délivrance de substances actives à la dispositif (1) est couplé à la dispositif (1) est.

13. Système selon l'une quelconque des revendications 11 à 12, **caractérisé en ce que** le dispositif (24) pourvue à son extrémité d'une sortie obturable (20) est , pendant le processus de remplissage le l'appareil mentionné (24) sur une télescopique broches (8) avec l'appareil (6) pour le remplissage du dispositif (1) est relié, en particulier avec à la télescopique broches (4) du dispositif (1).

14. Système selon l'une quelconque des revendications 11 à 13, **caractérisé en ce que** la station de recharge (9) est commandé par ordinateur, les station de recharge (9) communique par l'intermédiaire d'une interface ou d'une ordinateur central avec le dispositif (1), l'ordinateur (31) la station de recharge (9) comprend en particulier une moyen d'entrée et 1 ou une moyen l'sortie (32), en particulier avec d'un contact électrique ou un contact électro-mécanique de l'appareil (1) avec ladite station de recharge (9) la l'une automatique processus remplissage de la de l'appareil (1) démarre, en présence d'un correspondant signal de capteur le processus de remplissage se termine.

15. Système selon l'une quelconque des revendications 11 à 14, **caractérisé en, ce que** comprend la station de recharge (9) un appareil pour le vidage et / ou le nettoyage de l'appareil (1) et / ou la station de rechargement (9).
